(19) **Europäisches Patentamt**
**European Patent Office**
**Office européen des brevets**

(11) **EP 2 942 029 A1**

(12) **EUROPEAN PATENT APPLICATION**

(43) Date of publication:
**11.11.2015 Bulletin 2015/46**

(51) Int Cl.:
***A61B 19/00*** $^{(2006.01)}$

(21) Application number: **15166917.3**

(22) Date of filing: **08.05.2015**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA**

(30) Priority: **08.05.2014 KR 20140054783**

(71) Applicant: **Samsung Electronics Co., Ltd Gyeonggi-do 443-742 (KR)**

(72) Inventors:
- **Ahn, Sungwan**
  **Seoul (KR)**
- **Roh, Kyung Shik**
  **Gyeonggi-do (KR)**
- **Yoon, Sukjune**
  **Seoul (KR)**
- **Hwang, Hyoseok**
  **Seoul (KR)**

(74) Representative: **Grootscholten, Johannes A.M. et al**
**Arnold & Siedsma**
**Bezuidenhoutseweg 57**
**2594 AC The Hague (NL)**

(54) **SURGICAL ROBOTS AND CONTROL METHODS THEREOF**

(57)    A surgical robot may include: an image information acquisition unit configured to acquire image information of an intra-abdominal environment while the surgical robot performs a surgical operation; and/or a controller configured to recognize positions of an endoscope and a tool, mounted on the surgical robot, based on the acquired image information and kinematic information of links included in the endoscope and the tool. A surgical robot may include: an image information acquisition unit configured to acquire image information of an intra-abdominal environment while the surgical robot performs a surgical operation; an inertia measurement unit configured to acquire inertia measurement information of the surgical robot; and/or a controller configured to recognize positions of an endoscope and a tool, mounted on the surgical robot, based on the acquired image information and the inertia measurement information.

FIG. 10

## Description

BACKGROUND

1. Field

The invention relates to surgical robot and method of controlling said robot, specifically to methods of estimating positions of endoscopes and tools in real time based on endoscopic image information during surgical procedures using robots.

2. Description of Related Art

Minimally invasive surgery may generally refer to surgery capable of minimizing incision size, and laparoscopic surgery or surgery using surgical robots may have been used for minimally invasive surgery. Differently from laparotomy using a relatively large surgical incision through a part of a human body (e.g., the abdomen), in minimally invasive surgery, one or more small holes (incision holes or invasive holes) having a diameter, for example, of 0.5 centimeters (cm) to 1.5 cm may be formed through the abdominal wall, and an operator may insert an endoscope and surgical instruments through the one or more holes to perform surgery while viewing images provided by the endoscope.

Upon comparison with laparotomy, such minimally invasive surgery may cause less post-operative pain, may allow faster recovery of bowel movement, may allow earlier restoration of ability to eat, may allow shorter hospitalization, may allow faster return to daily life, and may promote better cosmetic effects owing to small incision size. Accordingly, minimally invasive surgery may have been used for cholecystectomy, prostatic calcinoma surgery, and hernia repair, etc., and applications thereof may continue to grow.

A surgical robot may include a master device, which may generate a required signal in accordance with manipulation of an operator (e.g., doctor) and may transmit the signal, and a slave robot, which may receive the signal from the master device and may directly perform manipulations required for surgery of a patient in response to signals received from the master device, even though the slave robot may be located far from the master device. In this regard, the master device may perform remote control of operations of the slave robot based on various physical information such as force, position, tactility, temperature, humidity, illuminance, and the like that may be detected by the slave robot.

In general, the slave robot may be installed in an operating room, and the master device may be installed in a manipulation room, and the master device and the slave robot may be connected to each other via wired or wireless communication to perform surgery at a distance. The doctor may be in the same room, in a different room, or in a different facility (perhaps located in another country).

Surgical robot systems may provide numerous other advantages, such as potentially improved precision, better ability to monitor the patient, and ability to record the surgical procedure for training, qualification, and evidentiary purposes.

When surgery is performed in the abdominal cavity by using a surgical robot, the operator may monitor information regarding an intra-abdominal environment via only an endoscope. However, a narrow viewing range of the endoscope may inhibit the operator from judging accurate positions of the endoscope and surgical tool(s) in the abdominal cavity during the surgery. This may be one of the reasons for interference or collision between the endoscope and tool(s), or damage of organs and tissues due to unnecessary movement of the endoscope and tool(s).

Although some example embodiments will be described with relation to surgical robots and methods of controlling those robots, those skilled in the art will appreciate that some example embodiments may be applied to other types of robots, such as robots not used in the medical field (e.g., aerospace robots, robots for handling hazardous materials, patrol robots, military robots), humanoid robots, or more general purpose systems and/or methods of controlling such systems.

SUMMARY

Some example embodiments may provide surgical robots capable of improving position recognition performance (accuracy and convergence of position recognition) by simultaneously recognizing a position of an endoscope and a position of a tool using not only position and orientation of the endoscope but also position and orientation of the tool as a state variable for a position recognition filter, and control methods thereof.

Some example embodiments may provide surgical robots capable of improving position recognition performance (accuracy and convergence of position recognition) by fusing kinematic information and various sensor information (endoscopic image information, inertia measurement information, and the like) during a position recognition process of an endoscope and a tool, and control methods thereof.

Some example embodiments may provide surgical robots capable of recognizing relative relationships between a modeled intra-abdominal environment and position/orientation of an endoscope and a tool by modeling the intra-abdominal environment based on position/orientation information of the endoscope and position information of feature points in the abdominal cavity obtained by a position recognition filter, and control methods thereof.

In some example embodiments, a method of controlling a surgical robot provided with an endoscope and a tool may comprise: acquiring image information regarding an intra-abdominal environment while the surgical robot performs a surgical operation; and/or recognizing positions of the endoscope and the tool based on the acquired image information and kinematic information of links included in the endoscope and the tool.

In some example embodiments, the method may further comprise: creating a map of the intra-abdominal environment based on results of the position recognition of the endoscope and the tool.

In some example embodiments, the recognizing of the positions of the endoscope and the tool may comprise: predicting positions and orientations of the endoscope and the tool, and a position of a feature point, based on currently acquired image information and the kinematic information; determining whether an existing landmark is identical to a feature point extracted from the currently acquired image information; and/or updating the predicted positions and orientations of the endoscope and the tool, and a position of a feature point registered as the landmark, by using the position of the existing landmark and position information of the feature point extracted from the currently acquired image information and matched with the existing landmark.

In some example embodiments, the method may further comprise: dividing the currently acquired image information into a plurality of regions of interest after predicting the positions and orientations of the endoscope and the tool, and the position of the feature point.

In some example embodiments, the dividing of the currently acquired image information into the plurality of regions of interest may comprise: calculating relative position and orientation information of the tool with respect to the endoscope by using the predicted positions and orientations of the endoscope and the tool; projecting a tool model onto the currently acquired image information; and/or dividing the currently acquired image information into a region of interest of a tool image and a region of interest of an intra-abdominal image.

In some example embodiments, a method of controlling a surgical robot provided with an endoscope and a tool may comprise: acquiring image information of an intra-abdominal environment and inertia measurement information of the surgical robot while the surgical robot performs a surgical operation; and/or recognizing positions of the endoscope and the tool based on the acquired image information and the acquired inertia measurement information.

In some example embodiments, the method may further comprise: creating a map of the intra-abdominal environment based on results of the position recognition of the endoscope and the tool.

In some example embodiments, the recognizing of the positions of the endoscope and the tool may comprise: predicting positions and orientations of the endoscope and the tool, and a position of a feature point, based on currently acquired image information and the inertia measurement information; determining whether an existing landmark is identical to a feature point extracted from the currently acquired image information; and/or updating the predicted positions and orientations of the endoscope and the tool, and a position of a feature point registered as the landmark, by using the position of the existing landmark and position information of the feature point extracted from the currently acquired image information and matched with the existing landmark.

In some example embodiments, the method may further comprise: dividing the currently acquired image information into a plurality of regions of interest after predicting the positions and orientations of the endoscope and the tool, and the position of the feature point.

In some example embodiments, the dividing of the currently acquired image information into the plurality of regions of interest may comprise: calculating relative position and orientation information of the tool with respect to the endoscope by using the predicted positions and orientations of the endoscope and the tool; projecting a tool model onto the currently acquired image information; and/or dividing the currently acquired image information into a region of interest of a tool image and a region of interest of an intra-abdominal image.

In some example embodiments, a surgical robot may comprise: an image information acquisition unit configured to acquire image information of an intra-abdominal environment while the surgical robot performs a surgical operation; and/or a controller configured to recognize positions of an endoscope and a tool, mounted on the surgical robot, based on the acquired image information and kinematic information of links included in the endoscope and the tool.

In some example embodiments, the controller may be further configured to create a map of the intra-abdominal environment based on results of the position recognition of the endoscope and the tool.

In some example embodiments, the controller may be further configured to recognize the positions of the endoscope and the tool by predicting positions and orientations of the endoscope and the tool, and a position of a feature point, based on currently acquired image information and the kinematic information, by determining whether an existing landmark is identical to a feature point extracted from the currently acquired image information, and by updating the predicted positions and orientations of the endoscope and the tool, and the position of a feature point registered as a landmark, by using the position of the existing landmark and position information of the feature point extracted from the currently acquired image information and matched with the existing landmark.

In some example embodiments, the controller may be further configured to divide the currently acquired image information into a plurality of regions of interest after predicting the positions and orientations of the endoscope and the tool, and the position of the feature point.

In some example embodiments, the controller may be further configured to divide the currently acquired image information into the plurality of regions of interest by calculating relative position and orientation information of the tool with respect to the endoscope by using the predicted positions and orientations of the endoscope and the tool, projecting a tool model onto the currently acquired image information, and dividing the currently acquired image information into a region of interest of a tool image and a region of interest of an intra-abdominal image.

In some example embodiments, a surgical robot may comprise: an image information acquisition unit configured to acquire image information of an intra-abdominal environment while the surgical robot performs a surgical operation; an inertia measurement unit configured to acquire inertia measurement information of the surgical robot; and/or a controller configured to recognize positions of an endoscope and a tool, mounted on the surgical robot, based on the acquired image information and the inertia measurement information.

In some example embodiments, the controller may be further configured to create a map of the intra-abdominal environment based on results of the position recognition of the endoscope and the tool.

In some example embodiments, the controller may be further configured to recognize the positions of the endoscope and the tool by predicting positions and orientations of the endoscope and the tool, and a position of a feature point, based on currently acquired image information and the inertia measurement information, by determining whether an existing landmark is identical to a feature point extracted from the currently acquired image information, and by updating the predicted positions and orientations of the endoscope and the tool, and a position of a feature point registered as a landmark, by using the position of the existing landmark and position information of the feature point extracted from the currently acquired image information and matched with the existing landmark.

In some example embodiments, the controller may be further configured to divide the currently acquired image information into a plurality of regions of interest after predicting the positions and orientations of the endoscope and the tool, and the position of the feature point.

In some example embodiments, the controller may be further configured to divide the currently acquired image information into the plurality of regions of interest by calculating relative position and orientation information of the tool with respect to the endoscope by using the predicted positions and orientations of the endoscope and the tool, projecting a tool model onto the currently acquired image information, and dividing the currently acquired image information into a region of interest of a tool image and a region of interest of an intra-abdominal image.

In some example embodiments, a surgical robot may comprise: a master device; and/or a slave robot configured to communicate with the maser device. The slave robot may be configured to acquire image information of an intra-abdominal environment while the surgical robot performs a surgical operation. The master device or the slave robot may be configured to recognize positions of an endoscope and a tool, mounted on the slave robot, based on the acquired image information and additional information.

In some example embodiments, the master device may be configured to recognize the positions of the endoscope and the tool, mounted on the slave robot, based on the acquired image information and the additional information.

In some example embodiments, the additional information may comprise kinematic information of links included in the endoscope and the tool.

In some example embodiments, the additional information may comprise inertia measurement information of the slave robot.

In some example embodiments, the master device may be further configured to create a map of the intra-abdominal environment based on results of the position recognition of the endoscope and the tool.

In some example embodiments, the slave robot may be configured to recognize the positions of the endoscope and the tool, mounted on the slave robot, based on the acquired image information and the additional information.

In some example embodiments, the additional information may comprise kinematic information of links included in the endoscope and the tool.

In some example embodiments, the additional information may comprise inertia measurement information of the slave robot.

In some example embodiments, the slave robot may be further configured to create a map of the intra-abdominal environment based on results of the position recognition of the endoscope and the tool.

In some example embodiments, the master device and the slave robot may be configured to recognize the positions of the endoscope and the tool, mounted on the slave robot, based on the acquired image information and the additional information.

BRIEF DESCRIPTION OF THE DRAWINGS

The above and/or other aspects and advantages will become more apparent and more readily appreciated from the following detailed description of example embodiments, taken in conjunction with the accompanying drawings, in which:

FIG. 1 is a perspective view illustrating an overall structure of a surgical robot according to some example embodiments;

FIG. 2 is an inner view illustrating portion PN of FIG. 1;

FIG. 3 is a block diagram illustrating a system for controlling a surgical robot according to some example embodiments;

FIG. 4 is a block diagram illustrating a system for controlling a surgical robot according to some example embodiments;

FIG. 5 is a diagram for describing a concept of a position recognition filter according to some example embodiments;

FIG. 6 is a diagram for describing a concept of calculating relative position information of a tool with respect to a coordinate system of a camera (e.g., endoscope) according to some example embodiments;

FIG. 7A is an image illustrating a result acquired by projecting a tool model onto an endoscopic image;

FIG. 7B is an image illustrating a result acquired by separating a tool image from an intra-abdominal image according to some example embodiments;

FIGs. 8A and 8B illustrate results of simultaneous position recognition of an endoscope and tools, intra-abdominal environment information, and relative distance according to some example embodiments;

FIG. 9 illustrates a result image acquired by registering abdominal cavity modeling information (e.g., endoscopic image) and pre-modeling information (e.g., diagnostic image) according to some example embodiments;

FIG. 10 is a flowchart illustrating a method of controlling a surgical robot according to some example embodiments; and

FIG. 11 is a flowchart illustrating an endoscopic image division and feature point extraction process of FIG. 10 according to some example embodiments.

## DETAILED DESCRIPTION

Example embodiments will now be described more fully with reference to the accompanying drawings. Embodiments, however, may be embodied in many different forms and should not be construed as being limited to the embodiments set forth herein. Rather, these example embodiments are provided so that this disclosure will be thorough and complete, and will fully convey the scope to those skilled in the art. In the drawings, the thicknesses of layers and regions may be exaggerated for clarity.

It will be understood that when an element is referred to as being "on," "connected to," "electrically connected to," or "coupled to" to another component, it may be directly on, connected to, electrically connected to, or coupled to the other component or intervening components may be present. In contrast, when a component is referred to as being "directly on," "directly connected to," "directly electrically connected to," or "directly coupled to" another component, there are no intervening components present. As used herein, the term "and/or" includes any and all combinations of one or more of the associated listed items.

It will be understood that although the terms first, second, third, etc., may be used herein to describe various elements, components, regions, layers, and/or sections, these elements, components, regions, layers, and/or sections should not be limited by these terms. These terms are only used to distinguish one element, component, region, layer, and/or section from another element, component, region, layer, and/or section. For example, a first element, component, region, layer, and/or section could be termed a second element, component, region, layer, and/or section without departing from the teachings of example embodiments.

Spatially relative terms, such as "beneath," "below," "lower," "above," "upper," and the like may be used herein for ease of description to describe the relationship of one component and/or feature to another component and/or feature, or other component(s) and/or feature(s), as illustrated in the drawings. It will be understood that the spatially relative terms are intended to encompass different orientations of the device in use or operation in addition to the orientation depicted in the figures.

The terminology used herein is for the purpose of describing particular example embodiments only and is not intended to be limiting of example embodiments. As used herein, the singular forms "a," "an," and "the" are intended to include the plural forms as well, unless the context clearly indicates otherwise. It will be further understood that the terms "comprises," "comprising," "includes," and/or "including," when used in this specification, specify the presence of stated features, integers, steps, operations, elements, and/or components, but do not preclude the presence or addition of one or more other features, integers, steps, operations, elements, components, and/or groups thereof.

Unless otherwise defined, all terms (including technical and scientific terms) used herein have the same meaning as commonly understood by one of ordinary skill in the art to which example embodiments belong. It will be further understood that terms, such as those defined in commonly used dictionaries, should be interpreted as having a meaning that is consistent with their meaning in the context of the relevant art and should not be interpreted in an idealized or overly formal sense unless expressly so defined herein.

Reference will now be made to example embodiments, which are illustrated in the accompanying drawings, wherein like reference numerals may refer to like components throughout.

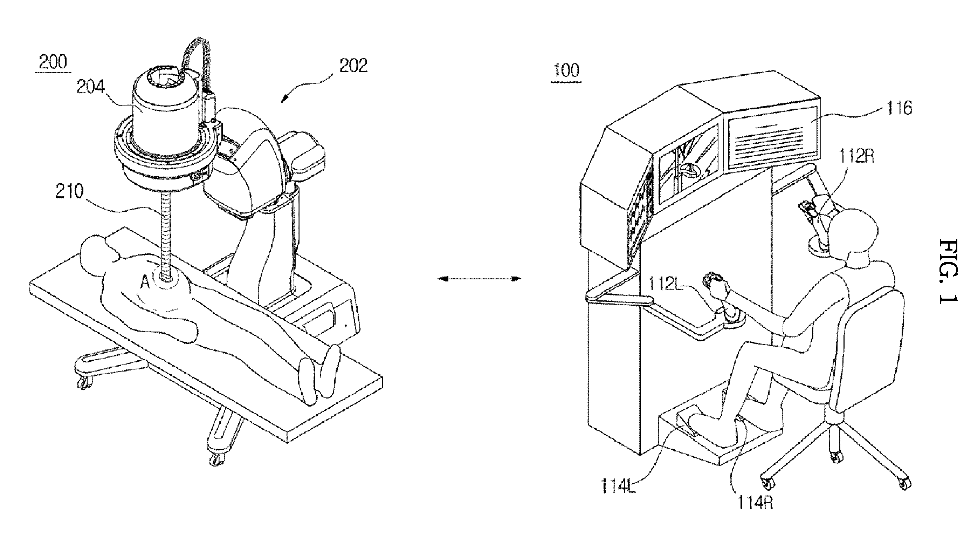

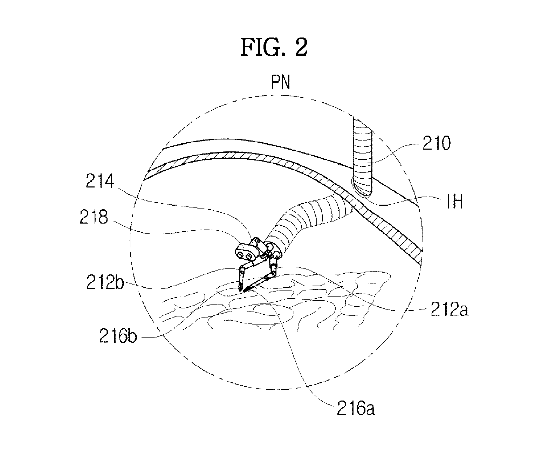

FIG. 1 is a perspective view illustrating an example of an overall structure of a surgical robot according to some example embodiments. FIG. 2 is an inner view illustrating portion PN of FIG. 1. Particularly, FIG. 1 illustrates a single-port surgical robot capable of performing surgery in various positions in a human body by introducing a plurality of surgical instruments, each provided with a surgical tool at a distal end thereof, into a patient's body through one incision hole (single-port). The following are several requirements for performing surgery by using a single-port surgical robot. First, the surgical instrument should have wide workspace since a plurality of surgical instruments are inserted into the human body through one incision hole and moved to a desired position for a surgical operation. Second, the surgical instrument should have high degree of freedom to perform various surgical operations, although collisions with tissues of the human body such as an abdominal wall are minimized. Third, information required for safe and precise surgery should be provided to an operator via visual guidance during surgery by using a slave robot having wide workspace and high degree of freedom.

As illustrated in FIG. 1, a surgical robot includes a slave robot 200 that performs surgery on a patient who lies on an operating table and a master device 100 that assists an operator (e.g., doctor) to remotely control the slave robot 200. The master device 100 generates a control signal in accordance with manipulation by the operator (e.g., doctor) and transmits the control signal to the slave robot 200. Meanwhile, the slave robot 200 receives the control signal from the master device 100 and moves in accordance with the received control signal to perform manipulation required for the surgery. In this regard, the master device 100 and the slave robot 200 are not necessarily separately arranged as physically independent devices, and may be integrated with each other as a single device.

In some example embodiments, the master device 100 may not be a single device, but may include more than one device, each performing one or more functions of the master device 100. Thus, in some example embodiments, the functionality of the master device 100 may be distributed.

Similarly, in some example embodiments, the slave robot 200 may not be a single robot, but may include more than one robot, each performing one or more functions of the slave robot 200. Thus, in some example embodiments, the functionality of the slave robot 200 may be distributed.

Therefore, in some example embodiments, the functionality of the master device 100, the slave robot 200, or the master device 100 and the slave robot 200 may be distributed.

In some example embodiments, the master device 100 may be required to perform certain functions, but may or may not perform other functions while maintaining its role as the master device 100. One or more of these other functions may be shared with or performed by the slave robot 200 (which maintains its role as the slave robot 200). Similarly, in some example embodiments, the slave robot 200 may be required to perform certain functions, but may or may not perform other functions while maintaining its role as the slave robot 200. One or more of those other functions may be shared with or performed by the master device 100 (which maintains its role as the master device 100).

Therefore, in some example embodiments, the required functionality of the master device 100 and the slave robot 200 may be maintained, while functionality that may be shared with or performed by the other robot may be so shared with or performed by the other robot consistent with the master device 100 maintaining its role as the master device 100 and the slave robot 200 maintaining its role as the slave robot 200.

As illustrated in FIGs. 1 and 2, the slave robot 200 may include a mounting arm 202 and a casing 204 (that may or may not be cylindrical).

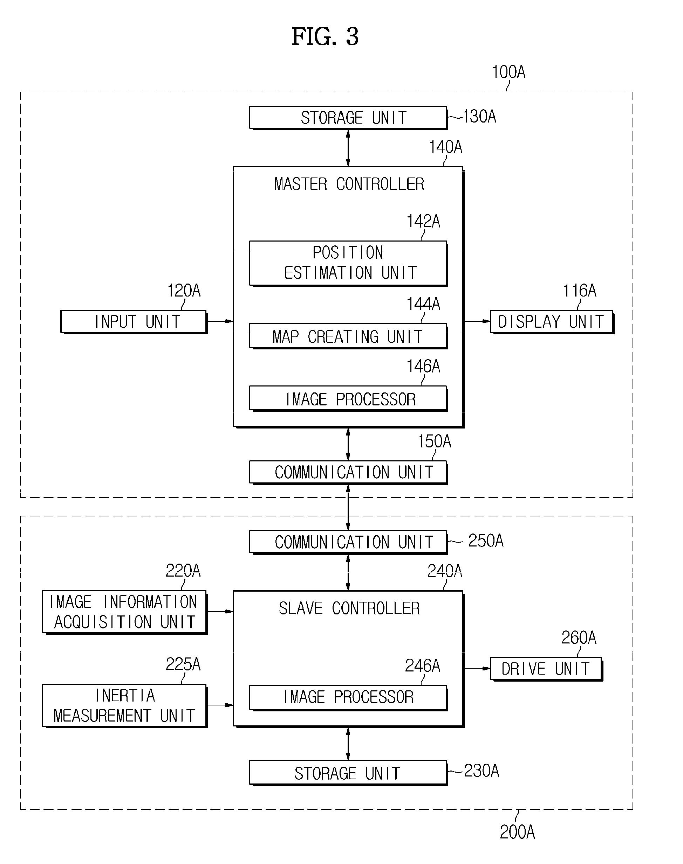

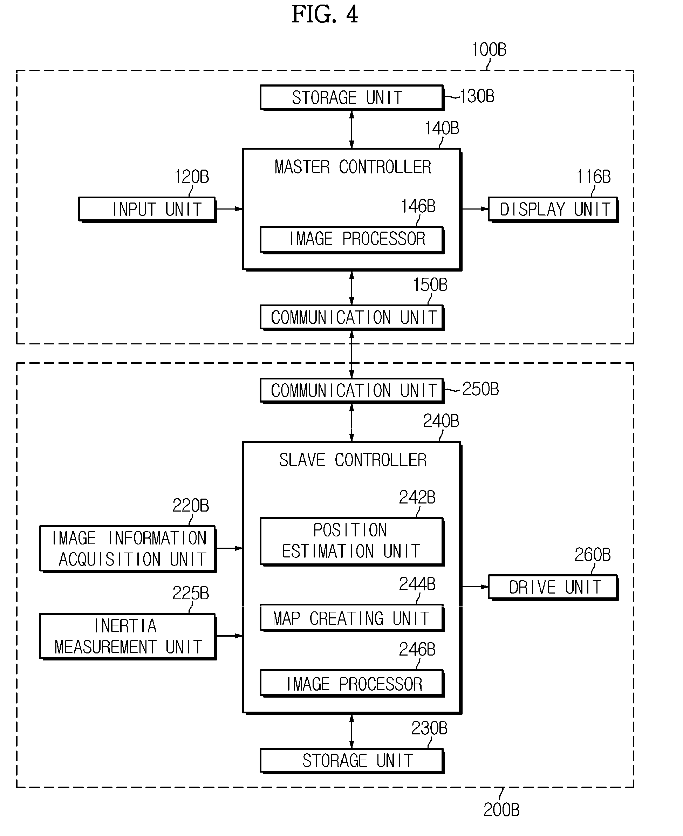

The mounting arm 202 of the slave robot 200 may be configured to be driven with multiple degrees of freedom. The mounting arm 202 includes a plurality of links and a plurality of joints, and an upper portion of the mounting arm 202 is connected to the casing 204. A guide tube 210 including a plurality of tools 212a and 212b, an endoscope 214, and a drive unit (260A of FIG. 3 and 260B of FIG. 4) for driving the plurality of tools 212a and 212b, the endoscope 214, and the guide tube 210 are embedded in the casing 204. The guide tube 210 is connected to the mounting arm 202 via the casing 204. When the slave robot 200 does not perform surgery, the guide tube 210 is embedded in the casing 204. While the slave robot 200 performs surgery, the guide tube 210 embedded in the casing 204 is brought out of the casing 204 and inserted into the patient's body as illustrated in FIGs. 1 and 2.

FIG. 2 illustrates that the guide tube 210 performs a surgical operation in a state of being inserted into the patient's body (e.g., inner view of portion PN of FIG. 1) in more detail. When the guide tube 210 is inserted into the patient's body through an incision hole IH formed on the patient's skin and approaches a target region for surgery (e.g., surgical region), the plurality of tools 212a and 212b and the endoscope 214 are branched off from the guide tube 210 and perform the surgical operation. In this regard, the guide tube 210, the plurality of tools 212a and 212b, and the endoscope 214 may also include a plurality of links and a plurality of joints to be driven with multiple degrees of freedom in the same manner as the mounting arm 202. A distal end of each of the plurality of tools 212a and 212b is provided with an end effector 216a and 216b, which is a surgical tool, such as forceps, jaw, grasper, scissors, stapler, cautery, and needle contacting an organ in the abdominal cavity and directly performing a surgical operation, for example, cutting and suturing. In addition, an endoscope camera 218, that acquires image information of an object to be observed in the abdominal cavity such as organs, tissues, and lesions, is mounted on the distal end of the endoscope 214. The endoscope 214 may include a variety of endoscopes for surgery such as a thoracoscope, an arthroscope, and a rhinoscope in addition to a laparoscope widely used in surgery by using a robot.

Meanwhile, the master device 100 may include master manipulators 112L and 112R, pedal sensors 114L and 114R, and a display unit 116. Master manipulators 112L and 112R may facilitate surgical procedures by more than one doctor simultaneously.

The master device 100 includes the master manipulators 112L and 112R such that the operator controls the master manipulators 112L and 112R while gripping them with both hands. The operator manipulates positions and functions of the mounting arm 202, the guide tube 210, the plurality of tools 212a and 212b, and the endoscope 214 of the slave robot 200 via the master manipulators 112L and 112R. The master manipulators 112L and 112R may be configured to have 6 degrees of freedom to control x-axial, y-axial, and z-axial translational motions, and roll, pitch, and yaw directional rotational motions, of the mounting arm 202 and the like in three-dimensional (3D) space. The master manipulators 112L and 112R may be realized using two handles as illustrated in FIG. 1, and the control signal is transmitted to the slave robot 200 in accordance with the manipulation of the handles to control operation of the slave robot 200 including the mounting arm 202 and the like. The translational motions and rotational motions of the mounting arm 202, the guide tube 210, the plurality of tools 212a and 212b, and the endoscope 214 are performed via the manipulation of the handles, and the surgical operation such as suturing and insertion of a tube may be performed through such motions.

The master device 100 includes two pedal sensors 114L and 114R such that the operator steps on or presses the pedal sensors 114L and 114R with two feet to improve manipulation performance of the master manipulators 112L and 112R. An example of controlling operation of the mounting arm 202 by using the master manipulators 112L and 112R including two handles and the two pedal sensors 114L and 114R illustrated in FIG. 1 will be described in detail. First, position and operation of the mounting arm 202 may be controlled using a master manipulator 112L (left handle), and position and operation of the guide tube 210 may be controlled using a master manipulator 112R (left handle). In addition, while a mode switch (not shown) or button (not shown) included in the master device 100 is manipulated, the position of operation of a first tool 212a (left tool) may be controlled using the master manipulator 112L (left handle), and the position and operation of a second tool 212b (right tool) may be controlled using the master manipulator 112R (right handle). Furthermore, after the mode switch or button is manipulated and while a left pedal sensor 114L is manipulated, the position and operation of the endoscope 214 may be controlled by using the master manipulator 112L (left handle). In addition, after the mode switch or button is manipulated and while a right pedal sensor 114R is manipulated, the position and operation of the endoscope 214 may be controlled by using the master manipulator 112R (right handle).

FIG. 1 exemplarily illustrates that two master manipulators (handles) are mounted on the master device 100. However, a plurality of surgical instruments such as a guide tube and a plurality of tools may be manipulated in real time by adding a further handle thereto. The master manipulators 112L and 112R may have various mechanical configurations according to manipulation methods and may include various input units three-dimensionally moving and operating the mounting arm 202, the guide tube 210, and the plurality of tools 212a and 212b of the slave robot 200, such as a joystick. A plurality of links and a plurality of joints (e.g., connection portion between links) are connected to the master manipulators 112L and 112R. A rotation angle sensor (e.g., encoder), which detects a rotation angle of each joint connected to each of the master manipulators 112L and 112R, may be mounted on each of the plurality of the joints connected to the master manipulators 112L and 112R.

An image input by the endoscope 214 may be displayed on the display unit 116 of the master device 100 as a picture image. The display unit 116 may include at least one monitor displaying information required for surgery. For example, a plurality of monitors may support stereoscopic viewing or viewing from multiple angles at the same time. Although FIG. 1 exemplarily illustrates the display unit 116 as including three monitors, the number of the monitors may vary according to type or kind of information to be displayed.

The master device 100 and the slave robot 200 may be coupled to each other via a wired or wireless communication network and may transmit a control signal, an endoscopic image input through the endoscope 214, and detection information input by various sensors such as an inertia sensor to the other party (slave robot 200 or master device 100). When two control signals generated by two master manipulators (handles) provided at the master device 100 are required to be transmitted, the two control signal may be independently transmitted. For example, when a control signal to manipulate the position of the first tool 212a branched off from the guide tube 210 and a control signal to manipulate the position of the second tool 212b branched off from the guide tube 210 are required to be transmitted simultaneously or at similar time points, each of the control signals may be independently transmitted to the slave robot 200.

The independently transmitted control signals do not interfere with each other, and one control signal does not influence on the other control signal. In order to independently transmit a plurality of control signals as described above, various methods such as a method of transmitting control signals by adding header information to each control signal in a stage of generating the control signal, a method of transmitting control signals in accordance with generation order of the control signals, or a method of transmitting control signals by setting priority with respect to transmission order of each control signal may be used. In this case, interference between the control signals may be completely inhibited by independently forming transmission passages of respective control signals.

FIG. 3 is a block diagram illustrating an example of controlling a surgical robot according to some example embodiments.

As illustrated in FIG. 3, a surgical robot may include a master device 100A and a slave robot 200A.

The master device 100A may include an input unit 120A, a storage unit 130A, a master controller 140A, a communication unit 150A, and a display unit 116A.

The input unit 120A is a device allowing a user to input an operation instruction of the slave robot 200A (e.g., instruction to start surgery and instruction to perform surgical operation) and may include the aforementioned master manipulators 112L and 112R and pedal sensors 114L and 114R, a user interface UI, or the like.

The storage unit 130A is a memory to store pre-information and algorithms to allow the master controller 140A to recognize positions of the endoscope 214 and the plurality of tools 212a and 212b, and results of the position recognition. The storage unit 130A may store 3D model information (e.g., computer-aided design (CAD) model information) of each of the plurality of tools 212a and 212b, kinematic information (e.g., length information) of each of the links (e.g., structure connecting joints) respectively constituting the endoscope 214 and the plurality of tools 212a and 212b, results of position recognition of the endoscope 214 and the plurality of tools 212a and 212b during a surgical operation of the slave robot 200A calculated using a vision sensor-based simultaneous localization and mapping (SLAM) algorithm (e.g., position/orientation information of the endoscope, position/orientation information of the plurality of tools, and position information of a landmark), and a 3D map of an intra-abdominal environment created based on the result of position recognition. The storage unit 130A may also store a variety of diagnostic images such as an X-ray image, an ultrasonic image, a computed tomography (CT) scan image, and a magnetic resonance image (MRI) acquired before surgery.

The master controller 140A, which is a processor to control an overall operation of the surgical robot, may include a position estimation unit 142A, a map creating unit 144A, and an image processor 146A.

The position estimation unit 142A estimates a position/orientation of the endoscope 214 and positions/orientations of the plurality of tools 212a and 212b by applying the SLAM algorithm to image information acquired by the image information acquisition unit 220A of the slave robot 200A and kinematic information of each of the links constituting the endoscope 214 and the plurality of tools 212a and 212b, or by applying the SLAM algorithm to image information acquired by the image information acquisition unit 220A and inertia measurement information (e.g., acceleration information or angular velocity information) acquired by an inertia measurement unit 225A. The SLAM algorithm sets a position of a feature point in an image and position/orientation information of the endoscope 214 and the plurality of tools 212a and 212b as one state variable and simultaneously estimates elements constituting the state variable by stochastic filtering. This procedure includes a prediction process, a data association process, and an update process which are repeatedly performed. In this regard, examples of the stochastic filter may include Extended Kalman Filter, Particle Filter, and the like. In addition, the position estimation unit 142A may estimate the position/orientation of the endoscope 214 by using a vision sensor-based odometery.

The map creating unit 144A creates a 3D map of the intra-abdominal environment based on the results of position recognition performed by the position estimation unit 142A, such as position information and orientation information of the endoscope 214 and position information of the feature point of the intra-abdominal image.

The image processor 146A processes an image input from the image information acquisition unit 220A of the slave robot 200A (e.g., the endoscope camera 218 mounted on the distal end of endoscope 214), in order to output the input image as a picture image. In this regard, examples of the image processing may include magnification, reduction, rotation, translation, editing, and filtering of a captured image.

The communication unit 150A is a communication circuit connected to the master controller 140A and a communication unit 250A of the slave robot 200A via a wired or wireless communication network, and transmitting and receiving data. The communication unit 150A may transmit a torque control signal generated by the master controller 140A (e.g., a torque control signal corresponding to a joint torque to estimate a target rotation angle of each joint) to the slave robot 200A or receive image information (e.g., endoscopic image information) acquired by the image information acquisition unit 220A and inertia measurement information acquired by the inertia measurement unit 225A from the slave robot 200A.

The display unit 116A displays relative relationship between the intra-abdominal environment, which is modeled based on the result of position recognition performed by the position estimation unit 142A and the map created by the map creating unit 144A, and positions/orientations of the endoscope 214 and the plurality of tools 212a and 212b.

In addition, the display unit 116A outputs a picture image corresponding to an endoscopic image received from the image information acquisition unit 220A of the slave robot 200A (e.g., the endoscope camera 218 and/or various diagnostic images such as an X-ray image, an ultrasonic image, a computed tomography (CT) scan image, and a magnetic resonance image (MRI) acquired before surgery and stored in the storage unit 130A), as visual information.

The slave robot 200A directly performs manipulation required for surgery on the patient by operating the mounting arm 202, the guide tube 210, the plurality of tools 212a and 212b, and the endoscope 214 in accordance with the control signal received from the master device 100A. The slave robot 200A may include the image information acquisition unit 220A, the inertia measurement unit 225A, a storage unit 230A, a slave controller 240A, the communication unit 250A, and the drive unit 260A, as illustrated in FIG. 3.

The image information acquisition unit 220A is inserted into the patient's body and captures images of internal organs or a body cavity while moving, thereby acquiring image information of a surgical region. The image information acquisition unit 220A may be implemented using the endoscope 214. The image information acquired by the image information acquisition unit 220A may be transmitted to an image processor 246A of the slave controller 240A and undergo image processing, or may be transmitted to the master device 100A via the communication unit 250A without undergoing the image processing.

The inertia measurement unit 225A, that is a device for measuring a variety of navigation-related information of the slave robot 200A such as acceleration, velocity, and orientation (e.g., angle), is installed in the plurality of tools 212a and 212b and/or the endoscope 214 of the slave robot 200A and detects orientation information (e.g., angular information). The inertia measurement unit 225A generates roll, pitch, and yaw directional orientation information (e.g., angular information) by detecting a relative angle of the mounting arm 202 with respect to the gravity direction and an inertial system. The inertia measurement unit 225A includes a tilt sensor which measures angle and an angular velocity sensor which measures angular velocity. An accelerometer may be used as the tilt sensor, and a rate-gyroscope may be used as the angular velocity sensor.

The storage unit 230A stores information and algorithm(s) required for controlling operation of the slave robot 200A, information acquired by the slave robot 200A, and the like. For example, the storage unit 230A stores image information of a surgical region acquired by the image information acquisition unit 220A and inertia measurement information (e.g., acceleration information or angular velocity information) acquired by the inertia measurement unit 225A. The storage unit 230A may also store various diagnostic images such as an X-ray image, an ultrasonic image, a computed tomography (CT) scan image, and a magnetic resonance image (MRI) acquired before surgery.

The slave controller 240A, which is a processor for connecting various constituent elements forming the slave robot 200A and controlling operation of the slave robot 200A, transmits image information of the surgical region acquired by the image information acquisition unit 220A to the master device 100A via the communication unit 250A, or transmits the torque control signal, which is generated by the master controller 140A and received through communication unit 250A, to the drive unit 260A.

In addition, the slave controller 240A may include the image processor 246A, which processes an image of the surgical region acquired by the image information acquisition unit 220A. In this regard, examples of the image processing may include magnification, reduction, rotation, translation, editing, and filtering of a captured image. The image processing performed in the slave controller 240A may be omitted, if desired.

The communication unit 250A is a communication circuit connected to the slave controller 240A and a communication unit 150A of the master device 100A via a wired or wireless communication network, and transmitting and receiving data. The communication unit 250A may receive the torque control signal from the master device 100A or may transmit image information (e.g., endoscopic image information) acquired by the image information acquisition unit 220A and inertia measurement information (e.g., acceleration information or angular velocity information) acquired by the inertia measurement unit 225A to the master device 100A.

The drive unit 260A, which is an actuator, such as a motor to transmit electric power or hydraulic power to each of the plurality of joints constituting the mounting arm 202, the guide tube 210, the plurality of tools 212a and 212b, and the endoscope 214, rotationally drives each of the joints constituting the mounting arm 202, the guide tube 210, the plurality of tools 212a and 212b, and the endoscope 214 in accordance with the torque control signal received from the master controller 140A.

FIG. 4 is a block diagram illustrating another example of controlling a surgical robot according to some example embodiments.

As illustrated in FIG. 4, a surgical robot may include a master device 100B and a slave robot 200B.

The master device 100B may include an input unit 120B, a storage unit 130B, a master controller 140B, a communication unit 150B, and a display unit 116B.

The master controller 140B, which is a processor to control an overall operation of the surgical robot, may include an image processor 146B.

The slave robot 200B may include an image information acquisition unit 220B, an inertia measurement unit 225B, a storage unit 230B, a slave controller 240B, a communication unit 250B, and the drive unit 260B, as illustrated in FIG. 4.

A configuration of controlling the master device 100A and the slave robot 200A of the surgical robot according to some example embodiments of the present disclosure is described above with reference to FIG. 3. FIG. 3 illustrates that the master controller 140A of the master device 100A includes the position estimation unit 142A, which estimates the position and orientation of the endoscope 214 and the positions and orientations of the plurality of tools 212a and 212b, and the map creating unit 144A, which models intra-abdominal environment based on the result of position recognition performed by the position estimation unit 142A. Differently, according to some example embodiments, as illustrated in FIG. 4, the slave controller 240B of the slave robot 200B includes a position estimation unit 242B, a map creating unit 244B, and an image processor 246B.

Referring to FIG. 4, since the slave controller 240B estimates the positions of the endoscope 214 and the plurality of tools 212a and 212b, the storage unit 230B of the slave robot 200B may store information required to estimate the positions/orientations of the endoscope 214 and the plurality of tools 212a and 212b. For example, 3D model information of each of the plurality of tools 212a and 212b and kinematic information of each of the links respectively constituting the endoscope 214 and the plurality of tools 212a and 212b may be stored in the storage unit 230B. The storage unit 230B may also store the result of position recognition of the endoscope 214 and the plurality of tools 212a and 212b performed by the position estimation unit 242B (e.g., position/orientation information of the endoscope, position/orientation information of the plurality of tools, and position information of the landmark) and a 3D map of intra-abdominal environment created based on the result of position recognition. In this regard, the result of position recognition performed by the position estimation unit 242B of the slave controller 240B and the map of the intra-abdominal environment created based on the result of position recognition may be transmitted to the master controller 140B through the communication units 250B and 150B, and the master controller 140B displays a relative relationship between the intra-abdominal environment modeled based on the received result of position recognition, the map of the intra-abdominal environment, and the positions/orientations of the endoscope and the plurality of tools on a display unit 116B.

Since the control configuration of the surgical robot of FIG. 4 is different from the control configuration of FIG. 3, in that the slave controller 240B, instead of the master controller 140B, includes the position estimation unit 242B and the map creating unit 244B in FIG. 4, and the other constituent elements of the surgical robot of FIG. 4 are the same as those of the surgical robot of FIG. 3, detailed descriptions thereof will not be given herein.

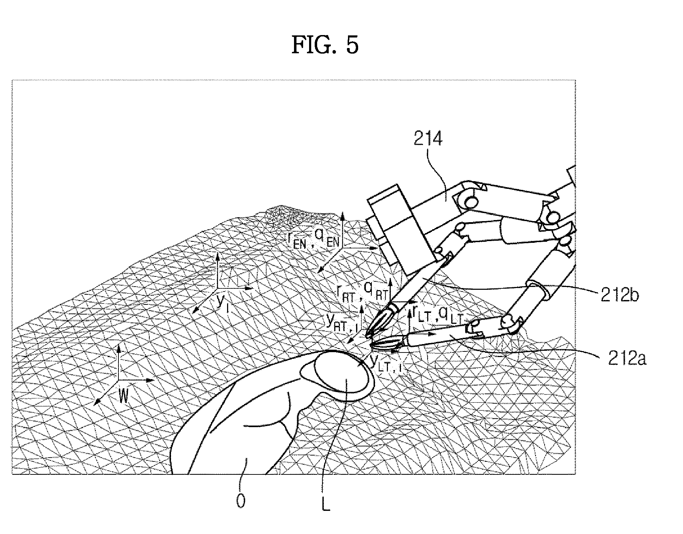

Hereinafter, a method of estimating (e.g., recognizing) positions/orientations of the endoscope and tools installed in the surgical robot and a method of creating a map of the intra-abdominal environment based on the recognition results will be described in detail with reference to FIGs. 5 to 10.

FIG. 5 is a diagram for describing a concept of a position recognition filter according to some example embodiments.

As illustrated in FIG. 5, the position recognition filter may estimate a position rEN and an orientation qEN of the endoscope 214, positions and orientations rLT, qLT, rRT, and qRT of the plurality of tools 212a and 212b, a position $y_1$ of a feature point of an intra-abdominal image, and positions yLT,i and yRT,j of feature points of a tool image with respect to the world coordinates W during a surgical procedure by using a robot in real time. In this regard, in FIG. 5, 'O' indicates organ, and 'L' indicates lesion.

The position recognition filter uses 3D positions 'r' and 3D orientations 'q' of the endoscope 214 and the plurality of tools 212a and 212b, and a 3D position 'y' of the feature point shown in an endoscopic image as one state variable x(k|k) and performs position estimation by using a stochastic filter algorithm (e.g., Kalman Filter and Particle Filter) with respect to the world coordinate system W. In some example embodiments, the 3D orientations of the endoscope 214 and the plurality of tools 212a and 212b are indicated as quaternion.

As illustrated in FIG. 5, when one endoscope 214 and two tools 212a and 212b are mounted in the slave robot 200, the position recognition filter may have the position and orientation rEN and qEN of the endoscope 214, the positions and orientations rLT, qLT, rRT, and qRT of the two tools 212a and 212b, positions $y_1$ of a plurality of feature points extracted from the intra-abdominal image, and positions yLT,i and yRT,j of a plurality of feature points extracted from the tool image, as elements of a state variable. The state variable x(k|k) may be represented by Equation 1 below.

[Equation 1]

$$x(k|k) = \begin{bmatrix} r_{EN}(k|k) \\ q_{EN}(k|k) \\ r_{LT}(k|k) \\ q_{LT}(k|k) \\ r_{RT}(k|k) \\ q_{RT}(k|k) \\ y_l(k|k) \\ y_{LT,i}(k|k) \\ y_{RT,j}(k|k) \end{bmatrix}$$

The position recognition filter used to estimate the positions/orientations of the endoscope 214 and the plurality of tools 212a and 212b, the positions $y_1$ of the plurality of feature points extracted from the intra-abdominal image, and the positions yLT,i and yRT,j of the plurality of feature points extracted from the tool image in real time includes a prediction stage and an update stage, each stage repeatedly performed.

Hereinafter, first, a process of predicting the positions and orientations rEN, qEN, rLT, qLT, rRT, and qRT of the endoscope 214 and the plurality of tools 212a and 212b will be described among elements of the state variable.

The predicting of the positions and orientations rEN, qEN, rLT, qLT, rRT, and qRT of the endoscope 214 and the plurality of tools 212a and 212b by the position recognition filter indicates estimating the state variable of a current stage through a motion model before fusing the positions and orientations rEN, qEN, rLT, qLT, rRT, and qRT of the endoscope 214 and the plurality of tools 212a and 212b with sensor information during the update stage. In this regard, the degree of movement of the endoscope 214 and the plurality of tools 212a and 212b from a previous stage 'k-1' to a current stage 'k' may be calculated as measurements in the form of relative change information in position and orientation 'δr' and 'δq' by using common kinematic information (e.g., length information) of each of the links respectively constituting the endoscope 214 and the plurality of tools 212a and 212b, or acceleration information or angular velocity information measured by the inertia measurement units 225A and 225B.

Meanwhile, in case of the endoscope 214 capable of using image information, relative change information in position and orientation 'δr' and 'δq' of the endoscope 214 may be calculated as measurements based on relationship between feature points acquired by extracting the feature points from each image using an image of the current stage 'k' and an image of the previous stage 'k-1' as inputs, and matching the feature points extracted from each of the images by vision sensor-based odometry.

A process of obtaining a predicted state variable from the relative change information in position and orientation is performed using a motion prediction model f(·). This process may be expressed by Equation 2 below.

[Equation 2]

$$x(k|k-1) = f\Big(x(k-1|k-1), \delta x(k)\Big)$$

Equation 2 is a general equation to calculate a predicted state variable by using the relative change information in position and orientation in the prediction stage of the position recognition filter. In this regard, x(k|k-1) indicates the predicted state variable, f(·) indicates the motion prediction model, x(k-1|k-1) indicates an updated state variable during the previous stage 'k-1', and δx(k) indicates relative change information in position and orientation.

Equation 2 may indicate a process of calculating a predicted state variable x(k|k-1) of the current stage by applying the motion prediction model f(·) to the updated state variable x(k-1|k-1) in the previous stage and the relative change information in position and orientation δx(k), which indicates the degree of movement from the previous stage 'k-1' to the current stage 'k'. The state variable 'x' of Equation 2 includes both of the position information and orientation information of the endoscope 214 and the plurality of tools 212a and 212b. In other words, δx(k) is a concept including relative change information in position 'δr' and relative change information in orientation 'δq'.

Predicted position information r(k|k-1) of the endoscope 214 and the plurality of tools 212a and 212b may be calculated using Equation 3 below and predicted orientation information q(k|k-1) of the endoscope 214 and the plurality of tools 212a and 212b may be calculated using Equation 4 below.

[Equation 3]

$$r(k|k-1) = r(k-1|k-1) + R[q(k-1|k-1)]\delta r(k)$$

In Equation 3, R is a rotation matrix.

[Equation 4]

$$q(k|k-1) = q(k-1|k-1) \times \delta q(k)$$

In addition, uncertainty that estimates the degree of error of the predicted state variable may be predicted based on a covariance matrix or particle distribution based on the model of Equation 2 in accordance with the stochastic filter algorithm.

When Kalman filter is used as the stochastic filter, the covariance matrix may be calculated based on Jacobian matrix F of the motion prediction model f(·) and noise Q(k) of the relative change information in position and orientation 'δr' and 'δq' as shown in Equation 5 below.

[Equation 5]

$$P(k|k-1) = FP(k-1|k-1)F^T + Q(k)$$

In some example embodiments, P(k|k-1) is a predicted covariance matrix, P(k-1|k-1) is a covariance matrix updated in the previous stage (k-1), and $F^T$ is a transposed matrix of Jacobian matrix F. Meanwhile, when a particle filter is used as the stochastic filter, the distribution of particles may be obtained by sampling particles based on the motion prediction model f(·).

Among the elements of the state variable, the other elements (positions of feature points of the intra-abdominal image and feature points of the tool image(s)), except for the positions and orientation rEN, qEN, rLT, qLT, rRT, and qRT of the endoscope 214 and the plurality of tools 212a and 212b, may be predicted as follows.

The position $y_1$ of the feature point extracted from the intra-abdominal image is predicted such that there is no relative change in position in a static environment (for example, when there is no motion of organs in the abdominal cavity) in the same manner as in a constant position model. This may be expressed by Equation 6 below.

[Equation 6]

$$\Delta y_1 = [0,0,0]^T$$

The position $y_1$ of the feature point extracted from the intra-abdominal image may be predicted using an abdominal physical property model such as a finite element method (FEM) or by reflecting random Brownian motion noise in a dynamic environment (for example, when there is motion of organs in the abdominal cavity). This may be expressed by Equation 7 below.

[Equation 7]

$$\Delta y_1 = [dx, dy, dz]^T$$

A process of calculating a predicted position information $y_1(k|k-1)$ from the calculated change information in position $\Delta y_1$ of the feature point extracted from the intra-abdominal image may be expressed by Equation 8 below.

[Equation 8]

$$y_1(k|k-1) = y_1(k-1|k-1) + \Delta y_1$$

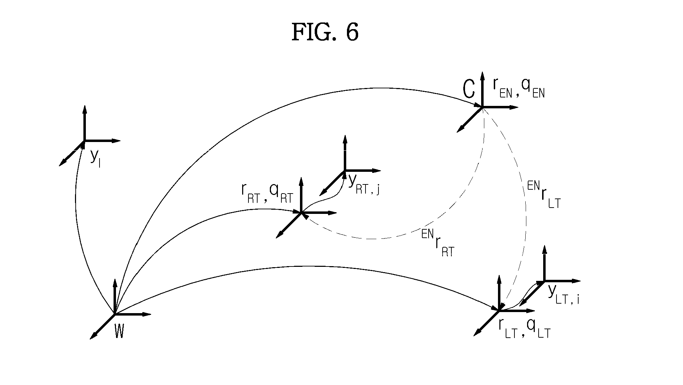

Meanwhile, the positions yLT,i and yRT,j of feature points extracted from the tool image may be predicted through 3D coordinate transforming of tool model information (e.g., positions of the feature points in the model), predicted position and orientation rEN and qEN of the endoscope 214, and predicted positions and orientations rLT, qLT, rRT, and qRT of the plurality of tools 212a and 212b, as illustrated in FIG. 6.

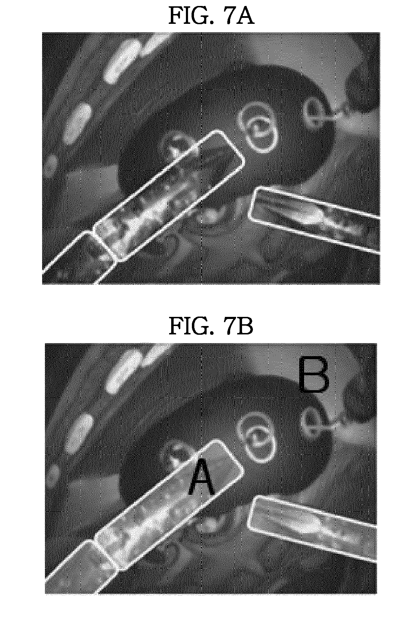

FIG. 6 is a diagram for describing a concept of calculating relative position information of a tool with respect to a coordinate system of a camera (e.g., endoscope) according to some example embodiments. FIG. 7A is an image illustrating a result acquired by projecting a tool model onto an endoscopic image. FIG. 7B is an image illustrating a result acquired by separating a tool image from an intra-abdominal image according to some example embodiments.

First, as illustrated in FIG. 6, relative position information of the plurality of tools 212a and 212b $^{EN}r_{LT}$ and $^{EN}r_{RT}$ with respect to a camera coordinate system C (e.g., dashed lines in FIG. 6) are obtained using information predicted in the prediction stage. Next, positions of feature points yLT,i and yRT,j extracted from the tool image are predicted by projecting the relative position information of the plurality of tools 212a and 212b $^{EN}r_{LT}$ and $^{EN}r_{RT}$ onto the camera coordinate system C through Equations 9 and 10 below using a camera model (e.g., intrinsic parameters $c_u$, $c_v$, $f_u$, and $f_v$ and radial distortion coefficients $k_1$ and $k_2$).

[Equation 9]

$$u_u = c_u + [f_u \cdot (^x/_z)]$$

$$v_u = c_v + [f_v \cdot (^y/_z)]$$

[Equation 10]

$$u_d = (u_u - c_u)(1 + k_1 r_u{}^2 + k_2 r_u{}^4) + c_u$$

$$v_d = (v_u - c_v)(1 + k_1 r_u{}^2 + k_2 r_u{}^4) + c_v$$

Here, $r_u = \sqrt{(u_u - c_u)^2 + (v_u - c_v)^2}$.

In some example embodiments, $(u_u, v_u)^T$ represents undistorted image coordinates, $(u_d, v_d)^T$ represents distorted image coordinates, $(c_u, c_v)^T$ represents coordinates in camera coordinate system C, $f_u$ and $f_v$ represent unit conversions from camera coordinate system C to world coordinate system W, $r_u$ represents the distance from coordinates $(c_u, c_v)^T$ in camera coordinate system C to undistorted image coordinates $(u_u, v_u)^T$, x, y, and z represent coordinates in world coordinate system W.

After performing the prediction stage of the positions/orientations of the endoscope 214 and the plurality of tools 212a and 212b, the positions of the plurality of feature points extracted from the intra-abdominal image, and the positions of the plurality of feature points extracted from the tool image, which are elements of the state variable, an endoscopic image division and feature point extraction stage is performed. In the endoscopic image division and feature point extraction stage, feature points repeatedly required in a subsequent update stage are extracted, and measurements and predicted measurements are properly matched. The endoscopic image division and feature point extraction stage is a process between the prediction stage and the update stage in the position recognition filter.

In the same manner as the method of predicting the positions $y_{LT,i}$ and $y_{RT,j}$ of the feature points extracted from the tool image, a model of the plurality of tools 212a and 212b (e.g., a region shown with white bold solid lines in FIG. 7A) may be projected onto the current endoscopic image by calculating the relative position information $^{EN}r_{LT}$ and $^{EN}r_{RT}$ of the plurality of tools 212a and 212b with respect to the endoscope 214 through Equations 9 and 10 as illustrated in FIG. 7A.

As illustrated in FIG. 7B, one region in which the projected tool model is located may be set as a region of interest (ROI) A of the tool image, and the other region may be set as an ROI B of the intra-abdominal image. Coordinates of the feature points respectively extracted from each of the ROIs are sequentially used as measurements in a subsequent update stage and are used to reduce errors in position recognition.

Mismatching of the feature points caused by movement of the plurality of tools 212a and 212b may be prevented by separating the tool image, which indicates one region occupied by the plurality of tools 212a and 212b, from the intra-abdominal image, which indicates the other region, except from the tool image, in the endoscopic image. Thus, performance of the position recognition filter may be improved.

The update stage of the position recognition filter may be performed using measurements z(k) obtained by sensor processing of the endoscopic image, noise measurements R(k), and predicted measurements h(k) obtained using the state variable x(k|k-1) acquired in the prediction stage. In this regard, the predicted measurements are obtained by transforming a 3D position of the feature points in the same manner as in Equations 9 and 10 with reference to a coordinate system of the endoscope (e.g., camera).

The feature points extracted from the tool image may be an artificial landmark, such as a marker attached to the plurality of tools 212a and 212b, or a natural landmark, such as an edge or a bolt hole of the plurality of tools 212a and 212b. On the other hand, the feature points extracted from the intra-abdominal image may be an image feature point, such as a corner or a blob, or human body model information such as a blood vessel, a nerve, or an organ.

The update stage of the position recognition filter may be differently applied thereto according to the stochastic filter algorithm such as Kalman filter and particle filter.

When Kalman filter is used as the stochastic filter, Kalman Gain K is calculated from Jacobian matrix H(k) of the predicted measurements h(k) and the noise measurements R(k) as shown in Equation 11 below, and a new estimated value and covariance matrix are calculated using Equations 12 and 13 blow, where $H(k)^T$ is a transposed matrix of Jacobian matrix H(k).

[Equation 11]

$$K = P(k|k-1)H(k)[H(k)P(k|k-1)H(k)^T + R(k)]^{-1}$$

[Equation 12]

$$x(k|k) = x(k|k-1) + K\big(z(k) - h(k)\big)$$

[Equation 13]

$$P(k|k) = \big(I - KH(k)\big)P(k|k-1)$$

Meanwhile, when a particle filter is used as the stochastic filter, an updated distribution of particles may be obtained by re-sampling particles obtained in the prediction stage by using a weight calculated from predicted measurements and actual measurements.

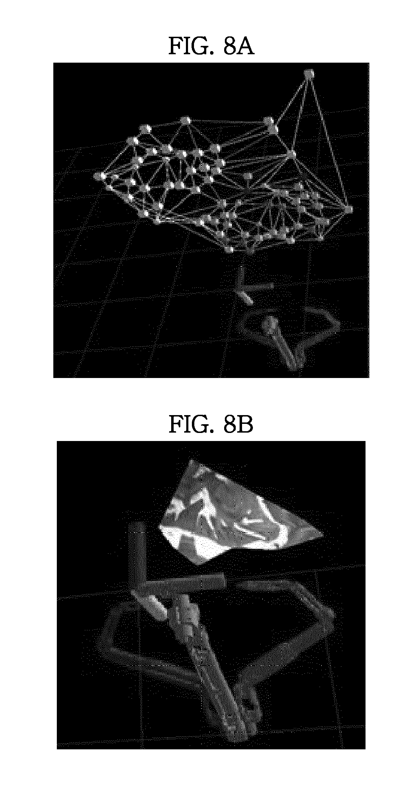

FIGs. 8A and 8B illustrate results of simultaneous position recognition of an endoscope and tools, intra-abdominal environment information, and relative distance according to some example embodiments.

After performing the position recognition process through the prediction stage, the endoscopic image division and feature point extraction stage, and the update stage, the intra-abdominal environment may be modeled based on positon/orientation information of the endoscope and position information of the feature points in the abdominal cavity which are the results of the position recognition filter.

Since the position recognition filter may estimate the positions $y_1$ of the feature points in the abdominal cavity in real time, the positions $y_1$ of the feature points may be expressed in a 3D space. As illustrated in FIG. 8A, the abdominal cavity may be three-dimensionally modeled by forming a triangular mesh using points in the 3D space.

When a stereo endoscope or an endoscope capable of calculating 3D distance information is additionally used, the intra-abdominal environment may be modeled as illustrated in FIG. 8B by registering 3D point cloud data by using an iterative closest point (ICP) algorithm or creating a probability-based grid map.

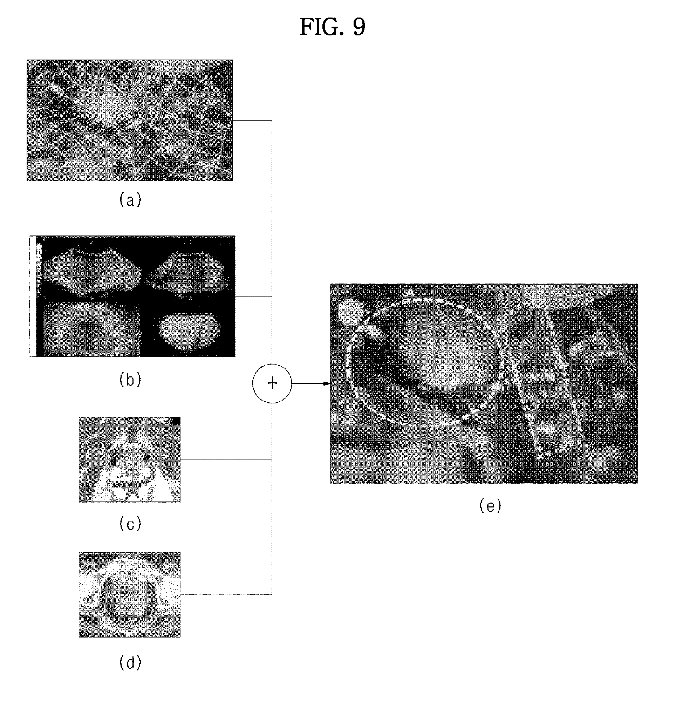

In addition, while the intra-abdominal environment may be modeled by using only the endoscopic image as illustrated in FIGs. 8A and 8B, a new result image may be generated by registering abdominal cavity modeling information (e.g., endoscopic image) and pre-modeling information (e.g., diagnostic image) according to some example embodiments, as illustrated in FIG. 9. In other words, as illustrated in FIG. 9, more detail and more precise modeling of the intra-abdominal environment may be performed by generating a resultant image (e) by registering a 3D endoscopic image (a), an ultrasonic image (b), a magnetic resonance (MR) image (c), and a CT image (d) with respect to a region to be operated on or examined.

The ICP algorithm used to register two pieces of 3D point cloud data uses an optimization method repeatedly performing a process, including aligning two pieces of closest 3D point cloud data defined as 'p' and 'q' and acquired by the stereo endoscope or the endoscope capable of calculating 3D distance information in one-to-one relationship, detecting a transformation minimizing a sum of the distance between, and detecting relationship in the transformed state. In this regard, by using Equation 14 below, the sum of the distance G(R, t) between 3D point cloud data 'q' and 3D point cloud data 'p' calculated via rigid transformation is used as a reference for the optimization method. Lastly, the rigid transformation relation (R', t') which minimizes Equation 14 is used for registration through Equation 15 below.

[Equation 14]

$$G(R, t) = \sum_i \| R p_i + t - q_i \|$$

[Equation 15]

$$(R', t') = \underset{R \in R_{3\times3}, t \in T_{3\times1}}{\arg\min} G(R, t)$$

Here, 'R' is rotation transformation matrix calculated as a result of performing the ICP algorithm, and 't' is a translational transformation matrix calculated as a result of performing the ICP algorithm.

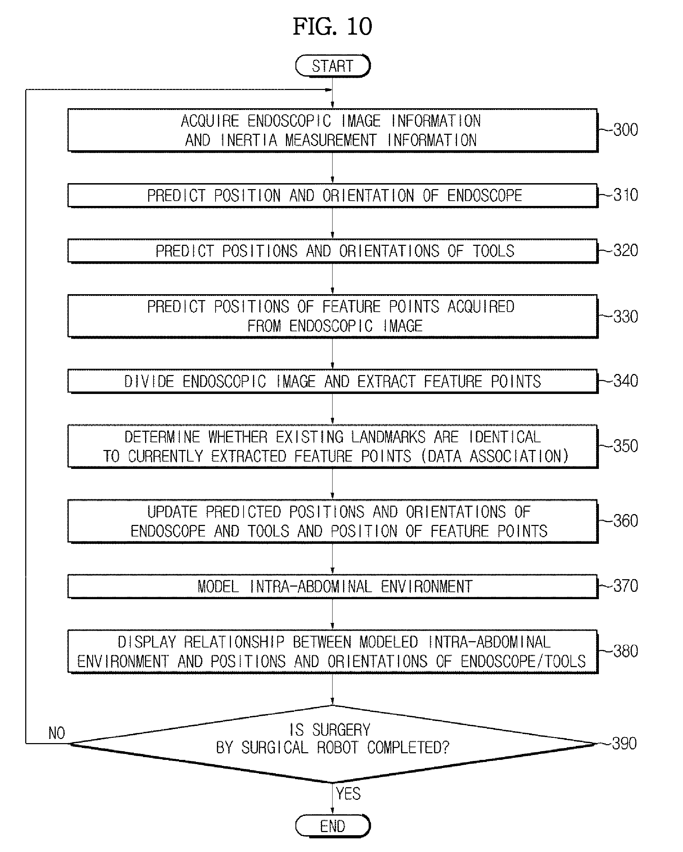

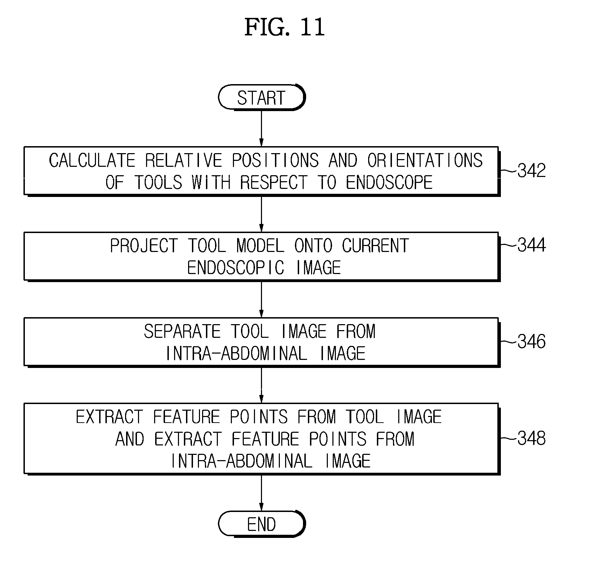

FIG. 10 is a flowchart illustrating a method of controlling a surgical robot according to some example embodiments. FIG. 11 is a flowchart illustrating an endoscopic image division and feature point extraction process according to some example embodiments.

As initial conditions for describing operations, the storage unit 130A stores 3D model information (e.g., CAD model information) of each of the plurality of tools 212a and 212b, kinematic information (e.g., length information) of each of the links (e.g., a structure connecting joints) respectively constituting the endoscope 214 and the plurality of tools 212a and 212b in advance as pre-information required to perform position recognition of the endoscope 214 and the plurality of tools 212a and 212b.

In addition, for descriptive convenience, a method of controlling a surgical robot will be described with reference to the master device 100A and the slave robot 200A illustrated in FIG. 3.

First, when a surgical operation instruction with regard to the surgical robot is input from the operator through the input unit 120A, surgery is initiated by the surgical robot.

When the surgical robot initiates surgery, the master controller 140A performs the surgical operation while periodically receiving image information of the intra-abdominal environment from the image information acquisition unit 220A of the slave robot 200A and inertia measurement information (e.g., acceleration information and angular velocity information) from the inertia measurement unit 225A (operation 300).

While the surgical operation is performed by the surgical robot, the position estimation unit 142A of the master controller 140A predicts a position and an orientation of the endoscope 214 based on a vision sensor-based SLAM algorithm and by additionally using kinematic information of each of the links constituting the endoscope 214 pre-stored in the storage unit 130A and inertia measurement information (e.g., acceleration information and angular velocity information) acquired by the inertia measurement unit 225A (operation 310). The position estimation unit 142A may also predict the position and orientation of the endoscope 214 by using vision sensor-based odometry.

Then, the position estimation unit 142A predicts positions and orientations of the plurality of tools 212a and 212b based on the vision sensor-based SLAM algorithm and by additionally using kinematic information of each of the links constituting the plurality of tools 212a and 212b pre-stored in the storage unit 130A and inertia measurement information (e.g., acceleration information and angular velocity information) acquired by the inertia measurement unit 225A (operation 320).

Then, the position estimation unit 142A predicts positions of feature points acquired from the endoscopic image (operation 330). Operations 320 and 330 correspond to a prediction stage of the SLAM algorithm.

Then, the position estimation unit 142A divides the endoscopic image acquired by the image information acquisition unit 220A into a tool image and an intra-abdominal image and extracts feature points respectively from the tool image and the intra-abdominal image (operation 340).

The endoscopic image division and feature point extraction process of operation 340 will be described in more detail. As illustrated in FIG. 11, the position estimation unit 142A calculates relative position and orientation information of the endoscope 214 and the plurality of tools 212a and 212b (operation 342).

Then, the position estimation unit 142A projects a tool model onto a current endoscopic image as illustrated in FIG. 7A (operation 344). In this regard, 3D model information (e.g., CAD model information) of each of the plurality of tools 212a and 212b, and the intrinsic parameters and the radial distortion coefficients of the endoscope 214 are used when the tool model is projected onto the endoscopic image.

Then, the position estimation unit 142A separates the tool image from the intra-abdominal image as illustrated in FIG. 7B (operation 346).

Then, the position estimation unit 142A extracts feature points respectively from the tool image and the intra-abdominal image (operation 348).

Referring back to FIG. 10, the position estimation unit 142A determines whether landmarks pre-stored in the storage unit 130A are identical to currently extracted feature points (operation 350). In other words, the position estimation unit 142A determines whether a feature point extracted from currently acquired image information is the same as a previously used landmark or whether the feature point should be registered as a new landmark through a tracking and matching process of the feature point. This process corresponds to a data association stage of the SLAM algorithm.

Then, the position estimation unit 142A updates the position and orientation of the endoscope 214 and the positions and orientations of the plurality of tools 212a and 212b, which are predicted in the prediction stage, and the positions of the feature points registered as the landmarks by using position information of the feature points previously registered as landmarks and position information of the feature points extracted from the currently acquired image information and matched with the previously registered landmarks (operation 360).

Then, the map creating unit 144A of the master controller 140A creates a map of the intra-abdominal environment based on position information and orientation information of the endoscope 214 and position information of the feature points of the intra-abdominal image which are results of position recognition performed by the position estimation unit 142A (operation 370).

Then, the master controller 140A displays relative relationships between the intra-abdominal environment modeled by the map creating unit 144A and the positions and orientations of the endoscope and tools by transmitting a control signal to the display unit 116A (operation 380).

Then, the master controller 140A determines whether the surgery of the surgical robot is completed (operation 390). The master controller 140A determines that the surgery of the surgical robot is completed when an instruction to stop the surgery of the surgical robot is input from the operator through the input unit 120A or rotation angle information is not received from the rotation angle sensor mounted on the master manipulators 112L and 112R for a desired time period (that may or may not be predetermined).

When the surgery of the surgical robot is not completed ('No' of operation 390), the master controller 140A returns to operation 300 and receives image information and inertia measurement information, and then predicts the positions and orientations of the endoscope and the tools by using information updated in the update stage (operations 310 and 320). Meanwhile, when the surgery of the surgical robot is completed ('Yes' of operation 390), the master controller 140A stores a final map of the intra-abdominal environment in the storage unit 130A and terminates the position recognition process of the endoscope and tools mounted on the surgical robot.

The method of FIG. 10 may be used in more general purpose systems and/or for methods of controlling such systems. For example, the method may be used in autonomous devices and/or for controlling such devices so as to allow operation of the autonomous devices.

The method of FIG. 11 may be used in more general purpose systems and/or for methods of controlling such systems. For example, the method may be used in aerospace robots and/or for controlling such robots so as to allow safe takeoff, movement, and/or landing of the robots.

As is apparent from the above description, according to the surgical robot and the control method thereof, position recognition performance (e.g., accuracy and convergence of position recognition) may be improved by simultaneously recognizing the position of the endoscope and the position of the tools using not only position and orientation of the endoscope but also positions and orientations of the tools as a state variable for a position recognition filter.

In addition, according to the surgical robot and the control method thereof, position recognition performance (e.g., accuracy and convergence of position recognition) may be improved by fusing kinematic information and various sensor information (e.g., endoscopic image information, inertia measurement information, and the like) during the position recognition process of the endoscope and the tools.

Furthermore, according to the surgical robot and the control method thereof, relative relationship between the intra-abdominal environment, which is modeled based on position/orientation information of the endoscope and position information of the feature points in the abdominal cavity obtained by a position recognition filter, and the positions and orientations of the endoscope and the tools in real time.

The algorithms discussed in this application (e.g., required to control the surgical robots and methods) may be used in more general purpose apparatuses and/or methods of controlling apparatuses. For example, the algorithms may be used in intelligent robots for handling equipment and materials and/or for controlling such intelligent robot so as to allow safe movement, packaging, and/or shipment of the equipment and materials.

The methods described above may be written as computer programs and can be implemented in general-use digital computers that execute the programs using a computer-readable recording medium. In addition, a structure of data used in the methods may be recorded in a computer-readable recording medium in various ways. Examples of the computer-readable recording medium include storage media such as magnetic storage media (e.g., ROM (Read-Only Memory), RAM (Random-Access Memory), USB (Universal Serial Bus), floppy disks, hard disks, etc.) and optical recording media (e.g., CD-ROMs (Compact Disc Read-Only Memories) or DVDs (Digital Video Discs)).

In addition, some example embodiments may also be implemented through computer-readable code/instructions in/on a medium (e.g., a computer-readable medium) to control at least one processing element to implement some example embodiments. The medium may correspond to any medium/media permitting the storage and/or transmission of the computer-readable code.

The computer-readable code may be recorded/transferred on a medium in a variety of ways, with examples of the medium including recording media, such as magnetic storage media (e.g., ROM, floppy disks, hard disks, etc.) and optical recording media (e.g., CD-ROMs or DVDs), and transmission media such as Internet transmission media. Thus, the medium may be such a defined and measurable structure including or carrying a signal or information, such as a device carrying a bitstream according to some example embodiments. The media may also be a distributed network, so that the computer-readable code is stored/transferred and executed in a distributed fashion. Furthermore, the processing element could include a processor or a computer processor, and processing elements may be distributed and/or included in a single device.

In some example embodiments, some of the elements may be implemented as a 'module'. According to some example embodiments, 'module' may be interpreted as software-based components or hardware components, such as a field programmable gate array (FPGA) or an application specific integrated circuit (ASIC), and the module may perform certain functions. However, the module is not limited to software or hardware. The module may be configured so as to be placed in a storage medium which may perform addressing, or to execute one or more processors.

For example, modules may include components such as software components, object-oriented software components, class components, and task components, processes, functions, attributes, procedures, subroutines, segments of program code, drivers, firmware, microcodes, circuits, data, databases, data structures, tables, arrays, and variables. Functions provided from the components and the modules may be combined into a smaller number of components and modules, or be separated into additional components and modules. Moreover, the components and the modules may execute one or more central processing units (CPUs) in a device.

Some example embodiments may be implemented through a medium including computer-readable codes/instructions to control at least one processing element of the above-described embodiments, for example, a computer-readable medium. Such a medium may correspond to a medium/media that may store and/or transmit the computer-readable codes.

The computer-readable codes may be recorded in a medium or be transmitted over the Internet. For example, the medium may include a ROM, a RAM, a CD-ROM, a magnetic tape, a floppy disc, an optical recording medium, or a carrier wave such as data transmission over the Internet. Further, the medium may be a non-transitory computer-readable medium. Since the medium may be a distributed network, the computer-readable code may be stored, transmitted, and executed in a distributed manner. Further, for example, the processing element may include a processor or a computer processor, and be distributed and/or included in one device.

Although some example embodiments have been shown and described, it would be appreciated by those skilled in the art that changes may be made in these example embodiments without departing from the principles and spirit of the example embodiments, the scope of which is defined in the claims and their equivalents. For example, while certain operations have been described as being performed by a given element, those skilled in the art will appreciate that the operations may be divided between elements in various manners.

Although some example embodiments are described above with relation to surgical robots and control methods thereof, those skilled in the art will appreciate that some example embodiments may be applied to other types of systems and methods, such as systems not used in the medical field (e.g., aerospace teleoperation systems and methods, apparatuses and methods for handling hazardous materials, patrol apparatuses and methods, military apparatuses and methods), humanoid apparatuses and methods, or more general purpose control systems and methods. Those skilled in the art will appreciate that the radiographic apparatuses and methods described in this application have a myriad of practical uses.

Although some example embodiments of the present disclosure have been shown and described, it would be appreciated by those skilled in the art that changes may be made in these example embodiments without departing from the principles and spirit of the disclosure, the scope of which is defined in the claims and their equivalents.

It should be understood that the example embodiments described herein should be considered in a descriptive sense only and not for purposes of limitation. Descriptions of features or aspects within each embodiment should typically be considered as available for other similar features or aspects in other embodiments.

**Claims**

1. A surgical robot (200), comprising:

   an image information acquisition unit (220A) configured to acquire image information of an intra-abdominal environment while the surgical robot performs a surgical operation;
   a mounting arm (202);
   an endoscope (204) mounted on the mounting arm (202);
   at least one tool (212a,212b) mounted on the mounting arm (202);
   wherein the endoscope and/or the at least one tool comprise a plurality of links and joints;
   an inertia measurement unit (225A) configured to acquire inertia measurement information of the surgical robot;
   a controller (140b,240b) configured to recognize positions of the endoscope and the at least one tool, based on the acquired image information and additional information, wherein the additional information comprises kinematic information of said links included in the endoscope and the tool and/or the acquired inertia measurement information.

2. The surgical robot according to claim 1, wherein the controller is further configured to create a map of the intra-abdominal environment based on results of the position recognition of the endoscope and the tool.

3. The surgical robot according to claim 1 or 2, wherein the controller is further configured to recognize the positions of the endoscope and the tool by predicting positions and orientations of the endoscope and the tool, and a position of a feature point, based on currently acquired image information and the kinematic information and/or inertia information, by determining whether an existing landmark is identical to a feature point extracted from the currently acquired image information, and by updating the predicted positions and orientations of the endoscope and the tool, and the position of a feature point registered as a landmark, by using the position of the existing landmark and position information of the feature point extracted from the currently acquired image information and matched with the existing landmark.

4. The surgical robot according to one of the previous claims, wherein the controller is further configured to divide the currently acquired image information into a plurality of regions of interest after predicting the positions and orientations of the endoscope and the tool, and the position of the feature point.

5. The surgical robot according to one of the previous claims, wherein the controller is further configured to divide the currently acquired image information into the plurality of regions of interest by calculating relative position and orientation information of the tool with respect to the endoscope by using the predicted positions and orientations of the endoscope and the tool, projecting a tool model onto the currently acquired image information, and dividing the currently acquired image information into a region of interest of a tool image and a region of interest of an intra-abdominal image.

6. Surgical robot as claimed in any of the preceding claims, wherein the inertia measurement unit (225A) comprises at least one sensor arranged in at least one of the plurality of tools (212a,212b) and/or the endoscope (214), wherein the at least one sensor is configured to determine the acceleration, velocity and orientation of the associated tool or endoscope, wherein the inertia measurement unit (225A) preferably is configured to determine roll, pitch and yaw directional orientation information relative to gravity direction and/or wherein inertia measurement unit (225A) comprises one or more tilt sensors and angular velocity sensors.

7. Surgical robot as claimed in any of the preceding claims, wherein the kinematic information comprises length information of the links of the endoscope and/or at least one tool.

8. A method of controlling a robot provided with an endoscope and at least one tool, preferably a surgical robot according to one of the claims 1-5, the method comprising:

   acquiring image information regarding an internal environment while the robot performs an operation;
   acquiring kinematic information and/or inertia information of the endoscope and/or the at least one tool;
   recognizing positions of the endoscope and the tool based on the acquired image information and kinematic information of links included in the endoscope and the at least one tool and/or based on the acquire image information and acquired inertia information .

9. The method according to claim 8, further comprising:

   creating a map of the internal environment based on results of the position recognition of the endoscope and the tool.

10. The method according to claim 8 or 9, wherein the recognizing of the positions of the endoscope and the tool comprises:

   predicting positions and orientations of the endoscope and the tool, and a position of a feature point, based on currently acquired image information and the kinematic information and/or acquired inertia information;
   determining whether an existing landmark is identical to a feature point extracted from the currently acquired image information; and
   updating the predicted positions and orientations of the endoscope and the tool, and a position of a feature point registered as the landmark, by using the position of the existing landmark and position information of the feature point extracted from the currently acquired image information and matched with the existing landmark.

11. The method according to one of the claims 8-10, further comprising:

   dividing the currently acquired image information into a plurality of regions of interest after predicting the positions and orientations of the endoscope and the tool, and the position of the feature point.

**12.** The method according to one of the claims 8-11, wherein the dividing of the currently acquired image information into the plurality of regions of interest comprises:

calculating relative position and orientation information of the tool with respect to the endoscope by using the predicted positions and orientations of the endoscope and the tool;
projecting a tool model onto the currently acquired image information; and
dividing the currently acquired image information into a region of interest of a tool image and a region of interest of an intra-abdominal image.

# FIG. 1

# FIG. 2

## FIG. 3

FIG. 4

100B

STORAGE UNIT — 130B

140B

MASTER CONTROLLER

120B

INPUT UNIT

146B

IMAGE PROCESSOR

116B

DISPLAY UNIT

150B

COMMUNICATION UNIT

COMMUNICATION UNIT — 250B

240B

SLAVE CONTROLLER

220B

IMAGE INFORMATION ACQUISITION UNIT

242B

POSITION ESTIMATION UNIT

225B

INERTIA MEASUREMENT UNIT

244B

MAP CREATING UNIT

260B

DRIVE UNIT

246B

IMAGE PROCESSOR

230B

STORAGE UNIT

200B

FIG. 5

FIG. 6

FIG. 7A

FIG. 7B

FIG. 8A

FIG. 8B

FIG. 9

(a)

(b)

(c)

(d)

(e)

## FIG. 10

START

ACQUIRE ENDOSCOPIC IMAGE INFORMATION
AND INERTIA MEASUREMENT INFORMATION — 300

PREDICT POSITION AND ORIENTATION OF ENDOSCOPE — 310

PREDICT POSITIONS AND ORIENTATIONS OF TOOLS — 320

PREDICT POSITIONS OF FEATURE POINTS ACQUIRED
FROM ENDOSCOPIC IMAGE — 330

DIVIDE ENDOSCOPIC IMAGE AND EXTRACT FEATURE POINTS — 340

DETERMINE WHETHER EXISTING LANDMARKS ARE IDENTICAL
TO CURRENTLY EXTRACTED FEATURE POINTS (DATA ASSOCIATION) — 350

UPDATE PREDICTED POSITIONS AND ORIENTATIONS OF
ENDOSCOPE AND TOOLS AND POSITION OF FEATURE POINTS — 360

MODEL INTRA-ABDOMINAL ENVIRONMENT — 370

DISPLAY RELATIONSHIP BETWEEN MODELED INTRA-ABDOMINAL
ENVIRONMENT AND POSITIONS AND ORIENTATIONS OF ENDOSCOPE/TOOLS — 380

NO — IS SURGERY
BY SURGICAL ROBOT COMPLETED? — 390

YES

END

FIG. 11

```
              ┌─────────────┐
              │    START    │
              └──────┬──────┘
                     │
                     ▼
  ┌─────────────────────────────────────────────┐
  │ CALCULATE RELATIVE POSITIONS AND ORIENTATIONS │~342
  │     OF TOOLS WITH RESPECT TO ENDOSCOPE        │
  └─────────────────────┬─────────────────────────┘
                        │
                        ▼
  ┌─────────────────────────────────────────────┐
  │       PROJECT TOOL MODEL ONTO CURRENT         │~344
  │            ENDOSCOPIC IMAGE                   │
  └─────────────────────┬─────────────────────────┘
                        │
                        ▼
  ┌─────────────────────────────────────────────┐
  │          SEPARATE TOOL IMAGE FROM             │~346
  │           INTRA-ABDOMINAL IMAGE               │
  └─────────────────────┬─────────────────────────┘
                        │
                        ▼
  ┌─────────────────────────────────────────────┐
  │    EXTRACT FEATURE POINTS FROM TOOL IMAGE     │
  │     AND EXTRACT FEATURE POINTS FROM           │~348
  │           INTRA-ABDOMINAL IMAGE               │
  └─────────────────────┬─────────────────────────┘
                        │
                        ▼
                 ┌─────────────┐
                 │     END     │
                 └─────────────┘
```

Europäisches
Patentamt

European
Patent Office

Office européen
des brevets

# EUROPEAN SEARCH REPORT

Application Number

EP 15 16 6917

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2007/287992 A1 (DIOLAITI NICOLA [US] ET AL) 13 December 2007 (2007-12-13) * paragraphs [0030], [0046]; figures 1,3,4 * | 1-7 | INV. A61B19/00 |
| Y | US 2006/258938 A1 (HOFFMAN BRIAN D [US] ET AL) 16 November 2006 (2006-11-16) * paragraphs [0071] - [0088]; figures 3,4 * | 1-7 | |
| Y | US 2014/051986 A1 (ZHAO TAO [US] ET AL) 20 February 2014 (2014-02-20) * abstract; figures 1,4a * | 3,4 | |
| Y | US 2009/088634 A1 (ZHAO WENYI [US] ET AL) 2 April 2009 (2009-04-02) * paragraphs [0090], [0143], [0202]; figures 8-9C * | 2,5 | |
| A | US 2009/192524 A1 (ITKOWITZ BRANDON D [US] ET AL) 30 July 2009 (2009-07-30) * figure 7 * | 1 | TECHNICAL FIELDS SEARCHED (IPC) A61B |

The present search report has been drawn up for all claims

| Place of search | Date of completion of the search | Examiner |
|---|---|---|
| The Hague | 15 September 2015 | Mayer-Martenson, E |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 15 16 6917

This annex lists the patent family members relating to the patent documents cited in the above-mentioned European search report.
The members are as contained in the European Patent Office EDP file on
The European Patent Office is in no way liable for these particulars which are merely given for the purpose of information.

15-09-2015

| Patent document cited in search report | | | Publication date | Patent family member(s) | | | Publication date |
|---|---|---|---|---|---|---|---|
| US 2007287992 | A1 | | 13-12-2007 | EP | 2038712 | A2 | 25-03-2009 |
| | | | | US | 2007287992 | A1 | 13-12-2007 |
| | | | | US | 2013226197 | A1 | 29-08-2013 |
| | | | | US | 2015057677 | A1 | 26-02-2015 |
| | | | | WO | 2007146984 | A2 | 21-12-2007 |
| US 2006258938 | A1 | | 16-11-2006 | CN | 101222882 | A | 16-07-2008 |
| | | | | CN | 103211563 | A | 24-07-2013 |
| | | | | EP | 1893118 | A1 | 05-03-2008 |
| | | | | EP | 2687182 | A1 | 22-01-2014 |
| | | | | EP | 2687183 | A1 | 22-01-2014 |
| | | | | EP | 2687184 | A1 | 22-01-2014 |
| | | | | EP | 2687185 | A1 | 22-01-2014 |
| | | | | EP | 2687186 | A1 | 22-01-2014 |
| | | | | EP | 2689740 | A1 | 29-01-2014 |
| | | | | EP | 2745795 | A1 | 25-06-2014 |
| | | | | KR | 20080027256 | A | 26-03-2008 |
| | | | | US | 2006258938 | A1 | 16-11-2006 |
| | | | | WO | 2006124388 | A1 | 23-11-2006 |
| US 2014051986 | A1 | | 20-02-2014 | CN | 104540439 | A | 22-04-2015 |
| | | | | EP | 2884879 | A1 | 24-06-2015 |
| | | | | KR | 20150043245 | A | 22-04-2015 |
| | | | | US | 2014051986 | A1 | 20-02-2014 |
| | | | | WO | 2014028394 | A1 | 20-02-2014 |
| US 2009088634 | A1 | | 02-04-2009 | US | 2009088634 | A1 | 02-04-2009 |
| | | | | US | 2012020547 | A1 | 26-01-2012 |
| | | | | US | 2015005622 | A1 | 01-01-2015 |
| US 2009192524 | A1 | | 30-07-2009 | CN | 102448680 | A | 09-05-2012 |
| | | | | EP | 2414137 | A2 | 08-02-2012 |
| | | | | JP | 2012521855 | A | 20-09-2012 |
| | | | | JP | 2014097431 | A | 29-05-2014 |
| | | | | KR | 20120004479 | A | 12-01-2012 |
| | | | | US | 2009192524 | A1 | 30-07-2009 |
| | | | | WO | 2010117685 | A2 | 14-10-2010 |

EPO FORM P0459

For more details about this annex : see Official Journal of the European Patent Office, No. 12/82